Europäisches Patentamt

European Patent Office

Office européen des brevets

(11) Veröffentlichungsnummer : **0 048 489**
**B1**

(12) **EUROPÄISCHE PATENTSCHRIFT**

(45) Veröffentlichungstag der Patentschrift :
02.12.87

(51) Int. Cl.⁴ : **A 61 B   5/10**

(21) Anmeldenummer : 81107538.1

(22) Anmeldetag : 22.09.81

(54) Vorrichtung zum Untersuchen eines Fingerreliefs.

(30) Priorität : 22.09.80 US 189176

(43) Veröffentlichungstag der Anmeldung :
31.03.82 Patentblatt 82/13

(45) Bekanntmachung des Hinweises auf die Patenterteilung : 02.12.87 Patentblatt 87/49

(84) Benannte Vertragsstaaten :
AT DE FR GB NL

(56) Entgegenhaltungen :
US-A- 3 614 737
US-A- 4 003 656
US-A- 4 053 228
US-A- 4 186 378
US-A- 4 236 082

(73) Patentinhaber : **Siemens Aktiengesellschaft Berlin
und München**
**Wittelsbacherplatz 2**
**D-8000 München 2 (DE)**

(72) Erfinder : **Rüll, Hartwig, Dr.**
**Balduin-Helm-Strasse 39**
**D-8080 Fürstenfeldbruck (DE)**

EP 0 048 489 B1

## Beschreibung

Die vorliegende Erfindung bezieht sich auf eine Vorrichtung zur Untersuchung eines Musters eines Fingers gemäß dem Oberbegriff des Anspruchs 1. Eine derartige Vorrichtung ist aus der Druckschrift US-A-4 003 656 bekannt.

Weitere Systeme zur Identifizierung von Fingerabdrücken, welche den Abdruck eines auf eine Kontaktfläche gedrückten Fingers identifizieren, sind bekannt.

Beispielsweise geht aus der US-PS 4 053 228 ein Gerät zur Identifizierung eines Fingers hervor, das eine transparente Glasplatte enthält, die als eine Kontaktfläche oder als ein Fingerabdruckleser dient. Ein Fingerabdruck wird dadurch gebildet, daß der zu untersuchende Finger auf die Rückfläche der Glasplatte gedrückt und in einer bestimmten Stellung darauf gehalten wird. Der Fingerabdruck wird von einem durch die Vorderfläche der Glasplatte gerichteten Lichtstrahl abgefragt. Der abfragende Strahl wird an der Rückfläche teilweise reflektiert und erzeugt einen Signalstrahl, der die Fingerabdruckinformation trägt. Der reflektierte Signalstrahl wird dann mit einem Hologramm des gleichen Fingerabdrucks korreliert, wodurch die Identifizierung der Person erhalten wird.

Aus der US-PS 4 120 585 geht ein anderes System zur Identifizierung eines Fingerabdrucks hervor. Dieses System enthält als Fingerabdrucksensor ein nachgiebiges oder elastisches optisches Prisma. Die Basis des Prismas wird vom Finger der zu untersuchenden Person berührt. Das nachgiebige Prisma verformt sich allgemein unter dem ausgeübten Druck. Es reflektiert teilweise einen abtastenden Lichtstrahl in Richtung einer fotoempfindlichen Einrichtung, die aktiviert wird. Die fotoempfindliche Einrichtung aktiviert ihrerseits weitere optische Komponenten des Systems zur Identifizierung von Fingerabdrücken. Ein Fingerabdruckleser wird auf das Muster der Erhöhungen und Vertiefungen, d. h. der Rippen und Furchen des Fingerabdrucks einer zu untersuchenden Person hin überprüft.

Die bekannten Systeme zur Identifizierung von Fingerabdrücken erfordern eine aufwendige Technik. Ein System zur Identifizierung eines Fingerabdrucks sollte jedoch leicht und billig herstellbar sein und gleichzeitig eine hohe Bildqualität des Fingerabdrucks liefern. Insbesondere sollte des Bild des Fingerabdrucks frei von Verzerrungen sein.

Die Aufgabe der Erfindung besteht darin, eine Vorrichtung zur Untersuchung eines Fingerabdruckmusters der genannten Art anzugeben, die leicht herstellbar ist und die ein verzerrungsfreies Bild hoher Qualität liefert.

Diese Aufgabe wird durch die im kennzeichnenden Teil des Patentanspruchs 1 angegebenen Merkmale gelöst.

Bevorzugte und vorteilhafte Ausführungsformen eines erfindungsgemäßen Fingerabdrucks gehen aus den Unteransprüchen 2 bis 10 hervor.

Eine erfindungsgemäße Vorrichtung weist folgende Vorteile auf. Sie wandelt die Fingerabdruckinformation eines Fingers in ein Ausgangssignal, speziell in ein elektrisches Ausgangssignal um. Das elektrische Ausgangssignal repräsentiert die in dem Fingerabdruck enthaltene Information zur Weiterverarbeitung in einen Rechner oder in ein anderes elektronisches Gerät eingelesen werden. Die Vorrichtung weist eine hohe Empfindlichkeit und ein hohes Auflösungsvermögen auf und arbeitet sehr zuverlässig. Sie wandelt die Fingerabdruckinformation unter Benutzung einer Abtasttechnik um.

Eine erfindungsgemäße Vorrichtung enthält eine Strahlungsquelle, die einen Strahl aussendet. Der Strahl kann aus elektromagnetischen Wellen oder aus Ultraschallwellen bestehen. Vorzugsweise wird Licht verwendet, wobei infrarotes, sichtbares oder ultraviolettes Licht in Frage kommt.

Die Vorrichtung weist auch eine den Finger abstützende Einrichtung oder ein Fingerbett auf. Wenn eine Untersuchung eines Fingers durchgeführt wird, wird dieser auf das Fingerbett gelegt. Das Fingerbett dient dazu, die Haut des Fingers frei von Verzerrungen zu halten, wenn das Muster des Fingers abgetastet wird. Damit der Strahl aus der Strahlungsquelle zur Haut des Fingers gelangen kann, weist das Fingerbett eine Öffnung oder ein Loch auf. Wenn der Finger für die Untersuchung auf dem Fingerbett ruht, wird der Strahl durch diese Öffnung direkt der Haut zugeführt. Anders ausgedrückt, die Öffnung oder das Loch läßt einen Bereich der Haut des Fingers zur direkten Bestrahlung und Abtastung frei.

Die erfindungsgemäße Vorrichtung weist des weiteren eine ortsfest angeordnete Fokussiereinrichtung auf, die den Strahl dem zu untersuchenden Bereich zuführt, sowie eine Abtasteinrichtung. Die Fokussiereinrichtung und die Abtasteinrichtung können zu einem einzigen Gerät oder Apparat kombiniert sein. Unter dem Einfluß der Fokussiereinrichtung erzeugt der Strahl einen Auftrefffleck auf dem zu untersuchenden Hautbereich.

Damit eine ausreichende Auflösung erhalten wird, muß die Ausdehnung des Flecks kleiner sein als der Abstand zwischen zwei benachbarten Rippen oder Furchen des Hautbereichs. Um einen kleinen Fleck auf der Fingerhaut zu erhalten, wird daher eine Fokussierungseinrichtung verwendet. Die Abtasteinrichtung führt den Auftrefffleck über den Hautbereich. Die Abtastung erfolgt Zeile um Zeile. Es ist möglich oder üblich, auf einer ersten Seite anzufangen und zur anderen oder zweiten Seite zu fahren, dann auf die nächste Zeile überzuwechseln und auf der zweiten Seite anzufangen und zur ersten Seite zu fahren. Die auftreffende Strahlung wird dem Muster der Erhöhungen und Vertiefungen der Haut entsprechend gestreut oder reflektiert. Wenn der Auftrefffleck die Abtastzeilen durchläuft, enthält die Intensität der zurückgestreuten oder -reflektierten Strahlung die Information über die Topologie der Haut des Fingers. Diese Information über den Finger wird durch elektrische Komponenten der erfindungsgemäßen Vorrichtung in ein elektrisches Ausgangssignal umgewandelt. Die erfindungsgemäße Vorrichtung weist des weiteren eine Detektoreinrichtung auf, die auf die zurückreflektierte oder -gestreute Strahlung anspricht. Sie enthält auch eine Abbildungseinrichtung, die den zurückgestreuten Strahl der Meßeinrichtung zuführt. In einer Ausführungsform, in der Licht als abtastende Strahlung benutzt wird, kann die Detektoreinrichtung ein Fotodetektor, beispielsweise eine CCD-Matrix oder eine Videokamera sein, und die zweite Abbildungseinrichtung kann eine Abbildungslinse oder ein abbildendes Linsensystem sein. Die Detektoreinrichtung mißt vorzugsweise die lokale Intensitätsverteilung der zurückgestreuten Strahlung. Das Ergebnis der Messung entspricht dem aus Rippen und Furchen bestehenden Muster des untersuchten Fingerbereichs. An einem Ausgang der Detektoreinrichtung ist ein entsprechendes Ausgangssignal entnehmbar. Dieses Signal, das vorzugsweise ein elektrisches Signal ist, kann in einem Rechner oder in einer anderen elektronischen Verarbeitungseinrichtung weiter verarbeitet werden. Das Ausgangssignal kann auch auf einem CRT-Schirm (Schirm einer Kathodenstrahlröhre) angezeigt werden.

Gemäß der Erfindung wird die Haut des Fingers direkt abgetastet, d. h. ohne ein zusätzliches Zwischenmaterial oder eine Zwischeneinrichtung. Wie oben dargelegt, wird die Abtastung mit einem Strahl einer geeignet gewählten Strahlung abgetastet. Die von der Haut des Fingers zurückgestreute Strahlung ist entsprechend der Topologie der Fingerhaut moduliert. Die zurückgestreute Strahlung enthält folglich alle Information über das Muster auf der Fingerspitze. Der zurückgestreute Strahl wird in ein elektrisches Ausgangssignal umgewandelt, das von einer Verarbeitungseinrichtung leicht weiterverarbeitet werden kann.

Bevorzugte Ausführungsformen der Erfindung werden anhand der Figuren in der folgenden Beschreibung näher erläutert. Von den Figuren zeigen :

Figur 1 in schematischer Darstellung eine erste erfindungsgemäße Ausführungsform,

Figur 2 ein Muster auf der Haut eines Fingers, das von einem Abtaststrahl erfindungsgemäß abgetastet wird,

Figur 3 in perspektivischer Darstellung den Aufbau eines rotierenden Polygonspiegels,

Figur 4 in perspektivischer Darstellung einen optischen Ablenker, der schrittweise um eine optische Achse drehbar ist, und

Figur 5 in schematischer Darstellung eine zweite Ausführungsform der Erfindung.

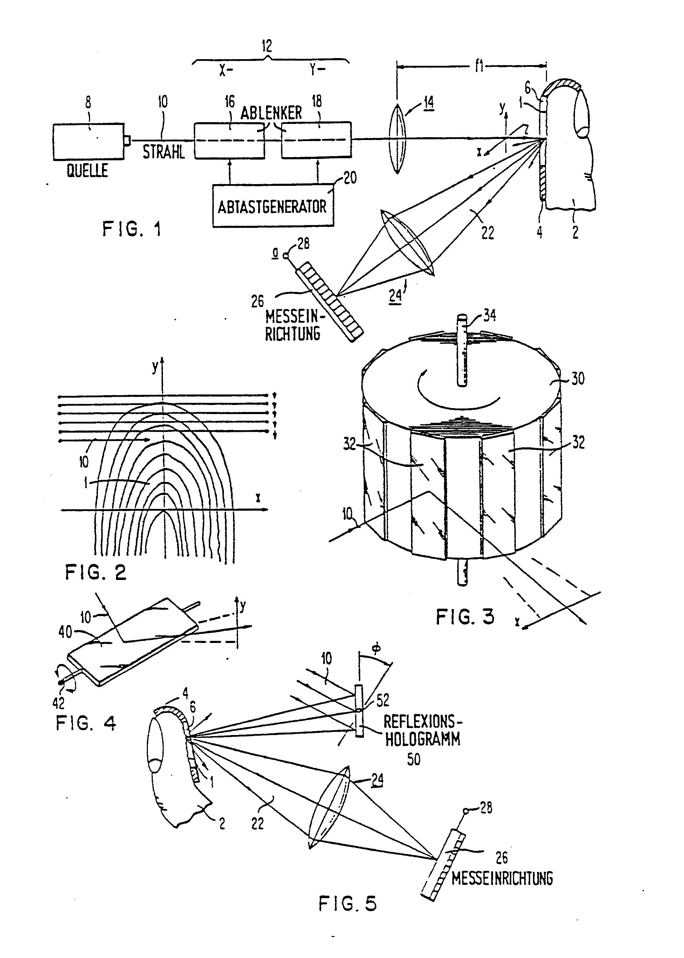

In der Figur 1 ist eine Vorrichtung zur Untersuchung des Hautmusters 1 der Kuppe eines Fingers 2 dargestellt. Die Vorrichtung weist ein geeignet konstruiertes Stützelement oder Fingerbett 4 auf, auf dem der Finger 2 ruhen kann. Des Fingerbett 4 weist eine Öffnung oder ein Loch 6 auf, welches den Bereich der Fingerhaut freiläßt, der untersucht werden soll, d. h. es ist kein Material vorgesehen, auf das der zu untersuchende Bereich des Fingers 2 während eines Abtastprozesses gedrückt wird. Deshalb kann ein weiter unten im einzelnen noch beschriebener Prüfstrahl frei durch das Loch 6 strahlen und hat dabei direkten Zugriff zur Fingerfläche 1. Das Fingerbett 4 ist dazu vorgesehen, während der Untersuchung den Finger 2 in einer fixierten Stellung zu halten. Da kein abdeckendes Material vorhanden ist, wird die Fingerfläche 1, d. h. das Muster der Erhöhungen und Vertiefungen der Haut dem prüfenden Strahl direkt ausgesetzt.

Die Vorrichtung umfaßt eine Quelle 8 zur Aussendung eines Strahls 10. Der Strahl 10 wird durch eine Abtasteinrichtung 12 und eine Strahlausrichtungseinrichtung 14 dem Hautmuster 1 zugeführt. Die Strahlausrichtungseinrichtung 14 bewirkt, daß der Strahl 10 auf dem zu untersuchenden Hautbereich einen kleinen Auftrefffleck erzeugt. Dieser Fleck weist einen Durchmesser auf, der kleiner ist als der Abstand zwischen zwei benachbarten Erhöhungen oder Vertiefungen auf der Haut.

Die Abtastvorrichtung 12 enthält einen x-Ablenker 16 zum Ablenken des Strahles 10 in einer x-Richtung und einen y-Ablenker 16 zum Ablenken des Strahles 10 in einer y-Richtung. Die Ablenker 16 und 18 sind hintereinander angeordnet. Die Richtungen x und y und die Ausbreitungsrichtung z des Strahles 10 sind senkrecht zueinander. Sowohl der x-Ablenker 16 als auch der y-Ablenker 18 werden von einem Abtastgenerator 20 gesteuert. Der Abtastgenerator 20 synchronisiert die x-Ablenkung bezüglich der y-Ablenkung.

Die Abtasteinrichtung 12 bewegt den Auftrefffleck über den zu untersuchenden Bereich 1. Die Folge davon ist, daß der Bereich 1 einen mit der Struktur oder dem Muster des zu untersuchenden Bereichs 1 modulierten Strahl 22 zurückstreut. Die gestreute Welle 22 wird über eine Strahlausrichtungseinrichtung 24 auf eine Abtast- oder Meßeinrichtung 26 gerichtet, die auf die von der Quelle 8 abgegebene Strahlung anspricht. Die Meßeinrichtung 26 mißt die Verteilung der zurückgestreuten Strahlung, die dem aus Erhöhungen und Vertiefungen bestehenden Muster des zu untersuchenden Bereichs 1 entspricht. Mit der Meßeinrichtung ist ein Ausgang 28 verbunden, an dem ein elektrisches Ausgangssignal a entnehmbar ist.

Die Figur 2 zeigt, wie der Auftrefffleck des Hautmuster oder den Bereich 1 abtastet. Der Strahl 10 wird Zeile um Zeile in der x-Richtung abgelenkt und diese Bewegung wird durch den x-Ablenker 16 erzeugt. Eine Bewegung in der y-Richtung von Zeile zu Zeile wird von dem y-Ablenker 18 erzeugt. In jeder Zeile beginnt der Strahl 10 auf der linken Seite und bewegt sich auf die rechte Seite.

Zum Abtasten des Fingers kann eine Ultraschallstrahlung verwendet werden. In einer Ausführungsform, die Ultraschalltechniken verwendet, müssen die Einrichtungen 8, 16, 18, 14, 24 und 26 aus Ultraschallkomponenten bestehen.

Eine Ultraschallquelle und die Ablenkeinrichtungen 16 und 18 zum Abtasten des Fingers 2 mit einem Ultraschallstrahl 10 sind ebenso wie die anderen Ultraschallkomponenten 14, 24 und 26 im Handel erhältlich.

Vorzugsweise besteht der abtastende Strahl 10 jedoch aus elektromagnetischer Strahlung. Es kann Infrarotlicht, sichtbares Licht oder Ultraviolettlicht benutzt werden. Insbesondere wird eine räumlich kohärente, monochromatische elektromagnetische Strahlung benutzt.

In einer im folgenden beschriebenen bevorzugten Ausführungsform besteht die Quelle 8 aus einem Laser, der einen monochromatischen und parallelen Lichtstrahl 10 aussendet. Der Lichtstrahl 10 wird durch eine als Abbildungslinse dargestellte Strahlausrichtungseinrichtung 14 auf die Haut des Fingers 2 fokussiert. Es kann auch ein zusammengesetztes Linsensystem zum Fokussieren benutzt werden. Die Abbildungslinse 14 ist in einem Abstand $f_1$ vom Finger 2 angeordnet. Dieser Abstand $f_1$ entspricht der Brennweite der Linse oder des Linsensystems 14. Deshalb wird der Laserstrahl 10 auf das Muster 1 fokussiert und dabei ein sehr kleiner Auftreffpunkt erhalten.

In der bevorzugten optischen Ausführungsform der Vorrichtung wird das zurückgestreute Licht 22 von einer geeignet ausgebildeten Abbildungseinrichtung empfangen, die als die zweite Strahlausrichtungseinrichtung 24 benutzt wird. Die Abbildungseinrichtung kann eine Linse, ein Hologramm, einen geeignet gekrümmten Fresnelschen Zonenspiegel oder dergleichen enthalten. Die Abbildungseinrichtung 24 erzeugt auf der Meßeinrichtung 26 ein Bild des Fingerbereichs 1.

Die Meßeinrichtung 26 kann einen Fotodetektor enthalten, der einen lichtempfindlichen Bereich zum Messen der Verteilung des auf ihn auftreffenden Lichts aufweist. Der lichtempfindliche Bereich kann ein einzelner Sensorbereich sein oder er kann aus einer Anordnung von Sensorelementen bestehen. Insbesondere kann eine CCD-Anordnung oder eine Videokamera benutzt werden. Das auf den lichtempfindlichen Bereich der Meßeinrichtung 26 fokussierte gestreute Licht wird entsprechend der Intensitätsverteilung in das elektrische Ausgangssignal a umgewandelt. Die Meßeinrichtung 26 kann die in dem Ausgangssignal a enthaltene Information einer nicht dargestellten weiteren Verarbeitungselektronik zur weiteren Verarbeitung zuführen.

Wie erwähnt, kann die Quelle 8 aus einem Laser bestehen. Dieser kann ein HE-Cd-Laser, ein He-Ne-Laser oder ein Halbleiterlaser sein.

Die Ablenker 16 und 18 können jeweils aus einem Galvanometerspiegel, einem akkustooptischen Strahlablenker, einem elektrooptischen Strahlablenker, einer Stimmgabel mit angebrachtem Spiegel, einem sich drehenden Spiegelpolygon, einem optomechanischen Strahlablenker, beispielsweise einem piezokeramischen Strahlablenker (siehe Appl. Opt., Vol. 15, S. 1432-1436, Juni 1976, Figur 5) usw. bestehen. Die verschiedenartigen Strahlablenker sind im Handel erhältlich.

Die erste Strahlausrichtungseinrichtung 14 kann eine fokussierende Linse sein. Sie kann beispielsweise eine einfache Glaslinse sein, die in einem ihrer Brennweite entsprechenden Abstand von dem Hautmuster 1 angeordnet ist. Die zweite Strahlausrichtungseinrichtung 24 kann eine Abbildungslinse sein. Beispielsweise kann sie eine einfache Glaslinse sein, die das Haut- oder Fingerabdruckmuster 1 verzerrungsfrei auf den lichtempfindlichen Bereich der Meßeinrichtung 26 abbildet.

Wie erwähnt, kann die Quelle 8 eine Ultraviolettquelle (UV-Quelle) sein. Wenn das von der menschlichen Haut auf den Finger 2 erzeugte Öl oder die darauf erzeugte Feuchtigkeit von einer UV-Quelle 8 beleuchtet wird, wird davon eine fluoriszierende Strahlung abgestrahlt, die zur Abbildung der Verteilung oder Struktur des Öl bzw. der Feuchtigkeit auf den lichtempfindlichen Bereich der Meßeinrichtung 26 benutzt werden kann.

In der Figur 3 ist ein optischer Ablenker zum Abtasten des Fingers 2 dargestellt. Der Lichtablenker weist einen rotierenden Zylinder 30 auf, dessen Mantel mit Spiegeln 32 versehen ist. Wenn der Zylinder 30 sich um seine Achse 34 dreht, wird der auftreffende Lichtstrahl 10 durch die Spiegel 32 immer wieder in der x-Richtung abgelenkt.

Gemäß Figur 4 kann der Ablenker ein Spiegel 40 sein, der um eine zur y-Richtung senkrecht stehenden Längsachse schrittweise gedreht wird. Der auftreffende Lichtstrahl 10 wird dadurch schrittweise in der y-Richtung abgelenkt.

In der Figur 5 ist eine andere Ausführungsform einer optischen Vorrichtung zum Überprüfen des Musters 1 eines Fingers 2 dargestellt. In dieser Ausführungsform kann der Lichtstrahl 10, der vorzugsweise ein Laserstrahl ist, durch ein und dasselbe Element fokussiert und gleichzeitig abgelenkt werden. Dieses Element besteht aus einem Reflexionshologramm 50. Die räumliche Orientierung des Hologramms kann entsprechend einem vorbestimmten Hautmuster geändert werden. Zu diesem Zweck ist das reflektierende Hologramm 50 mit einer Drehachse 52 versehen. Es sei darauf hingewiesen, daß der Lichtstrahl 10 auf das Fingermuster 1 in einem Winkel fokussiert wird, der nicht gleich dem Reflexionswinkel ist. Bei Verwendung eines Reflexionshologramms 50 kann eine Abbildungslinse 14 gemäß Figur 1 entfallen. Die gestreute Welle 22 wird in der gleichen Weise ausgewertet, wie in der Ausführungsform gemäß Figur 1.

## Patentansprüche

1. Vorrichtung zur Untersuchung des Musters auf einem Hautbereich (1) eines durch eine Fingerstützvorrichtung (4) gestützten Fingers (2), mit einer Abtasteinrichtung (12) zum linearen Abtasten des Hautbereichs mittels eines von einer Quelle (8) ausgesandten Strahls (10) einer elektromagnetischen oder Ultraschall-Strahlung, und mit einer relativ zur Quelle (8) ortsfesten strahlungsempfindlichen Detektoreinrichtung (26), der Strahlung (22) zuleitbar ist, die von dem Hautbereich (1) gestreut oder reflektiert ist, wobei die Abtasteinrichtung (12) eine in dem Strahl (10) angeordnete Strahlablenkeinrichtung (16, 18) aufweist, die den Strahl (10) über den Hautbereich (1) führt, und wobei sowohl die Stelle (8) als auch die Detektoreinrichtung (26) relativ zur Fingerstützvorrichtung (4) ortsfest angeordnet sind, gekennzeichnet durch eine relativ zur Fingerabstützeinrichtung (4) ortsfest angeordnete Fokussiereinrichtung (14) zum Fokussieren des Strahls (10) auf den Hautbereich (1) und durch eine Abbildungseinrichtung (24), die den Hautbereich (1) auf die Detektoreinrichtung (26) abbildet.

2. Vorrichtung nach Anspruch 1, dadurch gekennzeichnet, daß der Strahl (10) ein Lichtstrahl ist.

3. Vorrichtung nach Anspruch 2, dadurch gekennzeichnet, daß der Strahl (10) ein Laserstrahl ist.

4. Vorrichtung nach Anspruch 2 oder 3, dadurch gekennzeichnet, daß die Fokussiereinrichtung (14) aus einer Linse besteht.

5. Vorrichtung nach Anspruch 2, 3 oder 4, dadurch gekennzeichnet, daß die Detektoreinrichtung (26) einen Fotodetektor mit einem lichtempfindlichen Bereich zum Messen der auf ihn auftreffenden Lichtintensität aufweist.

6. Vorrichtung nach einem der vorhergehenden Ansprüche, dadurch gekennzeichnet, daß die Ablenkeinrichtung (16, 18) einen ersten und zweiten Strahlablenker (16 bzw. 18) aufweist, und daß der zweite Strahlablenker (18) den Strahl (10) in einer Richtung (y) ablenkt, die senkrecht zur Ablenkrichtung (x) des ersten Strahlablenkers (16) ist.

7. Vorrichtung nach Anspruch 6, dadurch gekennzeichnet, daß ein Strahlablenker (16, 18) einen drehbaren Polygonspiegel aufweist.

8. Vorrichtung nach Anspruch 6 oder 7, dadurch gekennzeichnet, daß ein Strahlablenker (16, 18) einen Galvanometerspiegel aufweist.

9. Vorrichtung nach einem der Ansprüche 6 bis 8, dadurch gekennzeichnet, daß ein Strahlablenker (16, 18) aus einem elektrooptischen Strahlablenker besteht.

10. Vorrichtung nach einem der Ansprüche 2 bis 9, dadurch gekennzeichnet, daß die Ablenkeinrichtung (16, 18) ein Reflexionshologramm aufweist, welches um eine Achse kippbar ist.

## Claims

1. A device for examining the pattern on a main zone (1) of a finger (2) supported by a finger support device (4), comprising a scanner, (12) which linearly scans the main zone by a beam (10) of electromagnetic or ultrasonic radiation transmitted from a source (8) a radiation-sensitive detector (26) which is stationary in relation to the source (8) and receives radiation (22) scattered or reflected at the main zone (1), where the scanner (12) comprises a beam-deflector (16, 18) arranged in the beam (10) to guide the beam (10) over the main zone (1), and where both the source (8) and the detector (26) are stationary relative to the finger support device (4), characterised by a focussing device (14) stationary relative to the finger support device (4) focusses the beam (10) onto the main zone (1), and a focussing device (24) which focusses the main zone (1) onto the detector (26).

2. A device as claimed in Claim 1, characterised in that the beam (10) is a light beam.

3. A device as claimed in Claim 2, characterised in that the beam (10) is a laser beam.

4. A device as claimed in Claims 2 or 3, characterised in that the focussing device (14) consists of a lens.

5. A device as claimed in Claims 2, 3 or 4, characterised in that the detector (26) comprises a photo-detector which has a light-sensitive zone for measuring the incident light intensity.

6. A device as claimed in one of the preceding Claims, characterised in that the deflector (16, 18) includes a first and second beam deflector (16 and 18 respectively), and that the second beam deflector (18) deflects the beam (10) in a direction (y) which is at right angles to the deflection direction (x) of the first beam deflector (16).

7. A device as claimed in Claim 6, characterised in that a beam deflector (16, 18) comprises a rotatable polygonal reflector.

8. A device as claimed in Claims 6 or 7, characterised in that a beam deflector (16, 18) comprises a galvanometer reflector.

9. A device as claimed in one of Claims 6 to 8, characterised in that a beam deflector (16, 18) is an electro-optical beam deflector.

10. A device as claimed in one of Claims 2 to 9, characterised in that the deflector (16, 18) comprises a reflection hologram which can be tilted about an axis.

## Revendications

1. Dispositif pour examiner le dessin présent dans une zone (1) de la peau d'un doigt (2) appuyé sur un dispositif (4) de soutien du doigt, et comportant un dispositif d'exploration (12) servant à explorer selon un balayage linéaire la zone de la peau à l'aide d'un faisceau (10), émis par une source (8) d'un faisceau électromagnétique ou d'un faisceau ultrasonore, et un dispositif de détection (26) sensible au rayonnement et fixe par rapport à la source (8) et auquel peut être envoyé un rayonnement (22), qui est diffusé ou réfléchi par la zone (1) de la peau, et dans lequel le dispositif d'exploration (12) possède un dispositif (7, 18) de déviation du faisceau, qui est disposé dans le faisceau (10) et qui dirige le faisceau (10) sur la zone (1) de la peau, et dans lequel aussi bien la source (8) que le dispositif de détection (26) sont disposés de manière à être fixes par rapport au dispositif (4) de support du doigt, caractérisé par un dispositif de focalisation (14) monté fixe par rapport au dispositif (4) de soutien du doigt et servant à focaliser le faisceau (10) sur la zone (1) de la peau, et par un dispositif de formation d'images (24), qui forme l'image de la zone (1) de la peau sur le dispositif de détection (26).

2. Dispositif suivant la revendication 1, caractérisé par le fait que le faisceau (10) est un faisceau de lumière.

3. Dispositif suivant la revendication 2, caractérisé par le fait que le faisceau (10) est un faisceau laser.

4. Dispositif suivant la revendication 2 ou 3, caractérisé par le fait que le dispositif de focalisation (14) est constitué par une lentille.

5. Dispositif suivant la revendication 2, 3 ou 4, caractérisé par le fait que le dispositif de détection (26) comporte un photodétecteur possédant une zone photosensible servant à mesurer l'intensité lumineuse tombant sur ce photodétecteur.

6. Dispositif suivant l'une des revendications précédentes, caractérisé par le fait que le dispositif de déviation (7, 18) comporte des premier et second éléments (16 ou 18) de déviation du faisceau et que le second élément (18) de déviation du faisceau dévie le faisceau (10) dans une direction y, qui est perpendiculaire à la direction de déviation (x) du premier élément (16) de déviation du faisceau.

7. Dispositif suivant la revendication 6, caractérisé par le fait qu'un élément (16, 18) de déviation du faisceau possède un miroir polygonal rotatif.

8. Dispositif suivant la revendication 6 ou 7, caractérisé par le fait qu'un élément (16, 18) de déviation du faisceau possède un miroir galvanomètre.

9. Dispositif suivant l'une des revendications 6 à 8, caractérisé par le fait qu'un élément (16, 18) de déviation du faisceau est constitué par un élément électrooptique de déviation du faisceau.

10. Dispositif suivant l'une des revendications 2 à 9, caractérisé par le fait que le dispositif déviateur (16, 18) comporte un hologramme à réflexion, qui peut pivoter autour d'un axe.

FIG. 1

FIG. 2

FIG. 3

FIG. 4

FIG. 5